# FASCICULE DE BREVET EUROPEEN

(11) **EP 3 888 792 B1**
(45) Date de publication et mention de la délivrance du brevet: **09.12.2026**
(21) Numéro de dépôt: 21163771.5
(22) Date de dépôt: 19.03.2021
(51) Int. Cl.: B03C 1/033, B03C 1/28, G01N 15/06, G01N 27/83

(54) **MODULE D'ATTRACTION ET DE DÉTECTION DE DÉBRIS**
MODUL ZUR ANZIEHUNG UND ERKENNUNG VON TRÜMMERTEILEN
MODULE FOR ATTRACTING AND DETECTING DEBRIS

(30) Priorité: 30.03.2020 BE 202005204
(43) Date de publication de la demande: 06.10.2021
(73) Titulaire: Safran Aero Boosters SA, 4041 Herstal (BE)
(72) Inventeur: RAIMARCKERS, Nicolas, Oscar, Louis, Gislain, 4041 Herstal (BE); MEUNIER, Aurélien, Guy, Edmond, Raoul, 4041 Herstal (BE); FRIPPIAT, Cédric, Louis, Marie, Ghislain, 4041 Herstal (BE); VALLINO, Frédéric, 4041 Herstal (BE)
(74) Mandataire: Ipsilon Luxembourg

(56) Documents cités:
- EP-A1- 2 455 774
- EP-A1- 3 363 518
- EP-A1- 3 441 753
- WO-A1-2017/157855
- CN-A- 107 907 040
- FR-A1- 3 085 995
- US-A- 4 841 244
- US-B1- 6 498 864

## Description

### Domaine technique

L'invention se rapporte à la surveillance d'organes mécaniques lubrifiés dans une turbomachine. Plus précisément, l'invention concerne la détection de débris ferromagnétiques dans l'huile d'une turbomachine. L'invention a également trait à une turbomachine, notamment un turboréacteur d'avion ou un turbopropulseur d'aéronef.

### Technique antérieure

La présence de débris métalliques dans l'huile d'un circuit de lubrification témoigne de l'usure des éléments mobiles d'une turbomachine. Ainsi, en analysant la quantité et la taille des débris métalliques en circulation dans l'huile, il est possible d'estimer la santé du moteur. En particulier, une augmentation brusque du nombre de débris détectés peut signifier qu'un roulement ou qu'un engrenage s'use prématurément. Dès lors, une maintenance doit être planifiée afin d'éviter une panne ou une casse mécanique.

Le document EP 3 363 518 A1 divulgue un système de détection électrique de présence de particules ferreuses dans un fluide. Ce système comporte à la fois un aimant et une bobine électrique. En fonctionnement, les particules ferreuses sont attirées par l'aimant et les perturbations du champ électro-magnétique sont mesurées pour en déduire la présence des débris. Pour éviter que les débris ne s'accumulent sur l'aimant, ce système est muni de crépines de protection de l'aimant. Les crépines permettent également de filtrer des débris de dimensions données pour qu'ils ne soient pas comptés par le détecteur.

Ce système présente une faiblesse car il peut générer des pertes de charge, négligeables pour des pressions et débits importants, mais qui peut altérer l'écoulement du fluide pour des flux de petite pression.

De plus, la fiabilité de la mesure présente une marge d'amélioration.

### Résumé de l'invention

### Problème technique

L'invention a pour objectif de résoudre au moins un des problèmes posés par l'art antérieur. Plus précisément, l'invention a pour objectif de proposer une alternative au système de détection de débris connus de l'état de la technique mais présentant une fiabilité de mesure supérieure, par le biais d'une attraction des particules ferromagnétiques accrue.

### Solution technique

L'invention a pour objet plusieurs modes de réalisation d'un module d'attraction et de détection de particules ferromagnétiques, chacun de ces modes de réalisation répondant au problème susmentionné.

L'invention porte ainsi sur un module d'attraction et de détection de débris ferromagnétiques dans un flux d'huile d'une turbomachine, le module comprenant : un aimant permanent ; et une bobine apte à détecter le champ magnétique généré par l'aimant ; le module étant remarquable en ce que l'aimant dispose d'une géométrie en H, définissant une branche centrale et deux branches latérales, la bobine étant enroulée autour de la branche centrale et les branches latérales formant chacune un des pôles de l'aimant.

L'attraction des particules est aussi améliorée par un module d'attraction et de détection de débris ferromagnétiques dans un flux d'huile d'une turbomachine, le module comprenant : un aimant permanent ; et une bobine apte à détecter le champ magnétique généré par l'aimant ; remarquable en ce que l'aimant comprend plusieurs paires de pôles.

L'attraction des particules est aussi améliorée par un module d'attraction et de détection de débris ferromagnétiques dans un flux d'huile d'une turbomachine, le module comprenant : un aimant permanent ; et une bobine apte à détecter le champ magnétique généré par l'aimant ; remarquable en ce que l'aimant est de forme cylindrique avec deux pôles séparés par un plan qui n'est ni parallèle à l'axe du cylindre, ni qui y est perpendiculaire. Dit autrement, l'axe Nord-Sud de l'aimant est incliné par rapport à l'axe défini par la forme cylindrique de l'aimant, cette inclinaison excluant la perpendicularité.

L'attraction des particules est aussi améliorée par un module d'attraction et de détection de débris ferromagnétiques dans un flux d'huile d'une turbomachine, le module comprenant: un aimant permanent; et une bobine apte à détecter le champ magnétique généré par l'aimant; remarquable en ce que l'aimant comprend plusieurs paires de pôles et en ce que les paires de pôles sont agencées sur l'axe de la bobine et séparées les unes des autres par un séparateur ferreux ou non ferreux.

Selon des modes de réalisation avantageux de l'invention, le module peut comprendre une ou plusieurs des caractéristiques suivantes, prises isolément ou selon toutes les combinaisons couvertes par les revendications jointes:
- la branche centrale est faite de fer doux ou de samarium-cobalt. Ces matériaux permettent d'amplifier le champ électro-magnétique de l'aimant et donc l'attraction des particules ferromagnétiques ainsi que le signal perçu par la bobine ;
- les paires de pôles sont agencées sur l'axe (A) de la bobine (72) et espacées les unes des autres. Ainsi, le champ électromagnétique « vu » par le flux est plus important sans nécessiter un aimant très encombrant ;
- les paires de pôles sont séparées deux à deux par un séparateur ferreux ou non ferreux, la bobine étant préférentiellement enroulée en partie autour du séparateur. Cette solution présente une bonne attraction des particules tout en limitant l'encombrement du module dans le flux ;
- l'aimant dispose d'une géométrie en H, définissant une branche centrale et deux branches latérales, la bobine étant enroulée autour de la branche centrale et les branches latérales formant chacune une paire de pôles ;
- les paires de pôles sont accolées. Ainsi, le pouvoir d'attraction de plusieurs aimants peut être combiné ;
- les paires de pôles sont toutes identiques, présentant chacune deux pôles axialement opposés, deux pôles diamétralement opposés, ou deux pôles opposés dans une direction qui n'est ni axiale ni diamétrale. Alternativement, les paires de pôles comprennent au moins deux paires de pôles distinctes, choisies parmi les paires de pôles présentant deux pôles axialement opposés, deux pôles diamétralement opposés, ou deux pôles opposés dans une direction qui n'est ni axiale ni diamétrale. Ces différentes variantes permettent d'optimiser le pouvoir d'attraction en fonction du débit d'huile ou de la configuration du canal dans lequel est agencé le module de détection de débris ;
- le module comprend en outre un barreau magnétique au contact de l'aimant et autour duquel est enroulée la bobine. Le barreau permet d'étendre le champ magnétique et donc le pouvoir d'attraction des particules présentes dans le flux d'huile ;
- le barreau est fait de plusieurs portions de barreau, intercalées entre deux paires de pôles. L'homogénéité du champ électromagnétique peut ainsi être améliorée au besoin ;
- le barreau a une extrémité distale biseautée. La surface externe du barreau et donc le pouvoir d'attraction est ainsi amélioré ;
- le barreau présente une surface striée ou crénelée, le barreau ayant notamment des tronçons de différents diamètres. Le ratio entre la surface externe du barreau et son volume peut ainsi être augmenté, rendant le pouvoir d'attraction plus important sans impact néfaste sur le volume et donc l'encombrement du module.

L'invention porte également sur un système de détection de débris ferromagnétiques dans un flux d'huile d'une turbomachine, le système comprenant un passage destiné à être parcouru par le flux et un module d'attraction et de détection des débris ferromagnétiques présents dans le flux, le module étant selon l'un des modes de réalisation évoqués ci-dessus, le module étant agencé dans le passage et comprenant en outre une enveloppe étanche au flux dans laquelle sont confinés l'aimant et la bobine. L'invention porte également sur un turboréacteur d'aéronef comprenant un groupe de lubrification fait d'un corps monobloc recevant plusieurs pompes et filtres, plusieurs entrées et sorties d'huile, et un système de détection de débris, le système de détection des débris étant selon l'un des modes de réalisation évoqués ci-dessus et le système de détection des débris étant disposé dans une entrée d'huile en amont des pompes et des filtres

L'enveloppe étanche permet de limiter les pertes de charge dans le flux d'huile qui seraient dues à la présence du module de détection.

L'enveloppe peut comprendre une cavité dans laquelle sont agencés l'aimant et la bobine. La bobine peut alternativement être noyée dans l'enveloppe et seul l'aimant est compris dans la cavité délimitée par l'enveloppe.

Il est entendu que le module peut comprendre une électronique de traitement du signal, intégrée à l'enveloppe, ou déportée et connectée avec ou sans fil au module. Le signal est traité en particulier pour détecter les variations du champ magnétique perçu par la bobine.

Par « passage », on entend un volume de l'espace parcouru par le fluide, qui peut être délimité par une paroi ou plusieurs parois permettant à un fluide de s'écouler selon au moins une direction.

Selon des modes de réalisation avantageux de l'invention, le système peut comprendre en outre une ou plusieurs des caractéristiques suivantes, prises isolément ou selon toutes les combinaisons techniquement possibles :
- le système comprend une crépine avec un treillis décrivant un cylindre ou une portion de cylindre et le module est intégré à la crépine, préférentiellement le module est disposé le long de l'axe du cylindre ou de la portion de cylindre. Cette intégration de la détection dans la crépine permet à la fois de limiter les pertes de charge et de limiter l'encombrement du dispositif entier ;
- la crépine est faite d'un matériau amagnétique. Son influence sur les mesures peut donc être limitée ;
- la crépine comprend une base et l'enveloppe est soudée ou sertie à la base de la crépine. Ce type d'assemblage permet d'assurer l'étanchéité de l'enveloppe, sans quoi une mesure imprécise ou des pertes de charge supplémentaires auraient lieu ;
- le barreau magnétique est un premier barreau magnétique, et le module comprend une seconde bobine agencée autour d'un second barreau magnétique, préférentiellement coaxial au premier barreau, le second barreau étant séparé du premier barreau par l'aimant et/ou par un séparateur en matériau polymère, isolant électromagnétiquement le premier barreau du second barreau. Cette subdivision du module permet de mesurer les particules à différents endroits du flux, par exemple pour des mesures redondantes renforçant la validité des mesures, ou pour des mesures dans différentes portions indépendantes du flux ;
- le barreau ou les barreaux est/sont cylindriques et sont positionnés coaxialement à l'enveloppe par une ou plusieurs entretoises de forme annulaire. Il est en effet important, notamment lors des mouvements de l'aéronef, de s'assurer que le barreau ne se déplace pas au sein de l'enveloppe. Alternativement aux entretoises un potting (colle) peut sceller la position du barreau ;
- le passage comprend au moins deux canaux permettant un écoulement du flux d'huile indépendamment dans les au moins deux canaux, et l'enveloppe s'étend dans les au moins deux canaux pour détecter les débris présents dans l'huile parcourant chacun des canaux, le module comprenant autant de bobines qu'il y a de canaux. Ceci permet la détection des débris indépendamment dans plusieurs circuits d'huile sans nécessiter plusieurs modules de détection. Un circuit peut par exemple lubrifier un roulement du turboréacteur alors qu'un autre circuit communique avec un échangeur de chaleur ;
- le module comprend un nombre égal d'aimants et de bobines, une bobine et un aimant étant prévu par canal traversé par l'enveloppe. Ceci permet des mesures complètement indépendantes. Alternativement, un aimant peut être commun à deux passages contigus pour minimiser le poids et la complexité du module ;
- le passage comprend au moins deux canaux contigus et séparés l'un de l'autre par une paroi, la paroi disposant d'un orifice qui est traversé par l'enveloppe et éventuellement par une crépine, l'aimant étant positionné au droit de cet orifice. Ainsi, l'aimant attire les débris vers la paroi et les bobines respectives dans les canaux sont à distance de la paroi ;
- un joint d'étanchéité est agencé entre l'enveloppe et la paroi et/ou entre l'enveloppe et la crépine, et/ou entre la crépine et la paroi. Ceci permet de s'assurer de l'étanchéité des flux entre les deux canaux contigus ;
- l'enveloppe comprend deux portions cylindriques de diamètres différents, la portion de plus gros diamètre recevant l'aimant et étant préférentiellement noyée dans une paroi du passage ou dans la base de la crépine. La portion de plus petit diamètre peut recevoir le barreau et la bobine entourée autour du barreau. Cette conception permet de maximiser la capacité de l'aimant à attirer les particules sans nécessiter la présence d'une enveloppe inutilement imposante dans le flux d'huile ;
- l'aimant et/ou le séparateur a/ont une rainure accueillant des câbles électriques reliés à la / aux bobine(s). Le passage des câbles est une difficulté supplémentaire de l'espace confiné de l'enveloppe. Une ou plusieurs rainures longitudinales selon l'axe de l'enveloppe permet le passage des câbles sans affecter la qualité de la détection ;
- le barreau a une extrémité distale située environ au centre du passage ou au centre d'un des canaux. L'extrémité du barreau est l'endroit attirant le plus de particules et pour augmenter la probabilité de capter toutes les particules en suspension dans l'huile, cette extrémité peut être située entre ¼ et ¾ du passage ;
- le passage comprend un coude dans lequel est agencé le module. Le flux est donc naturellement dirigé pour rencontrer le module et les particules ferromagnétiques sont dirigées par force centrifuge vers l'extérieur du coude où peut se trouver le module. Ceci augmente les chances que le module « voit » passer les particules. Le comptage des particules est donc rendu plus précis ;
- l'enveloppe est en saillie dans le passage d'une longueur qui est ajustable et/ou l'enveloppe est orientée transversalement à la direction principale d'écoulement du flux d'un angle qui est ajustable. Selon la vitesse du flux, la nature du flux, ou la géométrie du passage, cet ajustement permet d'obtenir une détection la plus précise possible ;
- l'aimant n'est pas disposé dans le passage ou dans un des canaux du passage. L'aimant peut ainsi être noyé dans une paroi du passage ou des canaux ;
- la bobine est un insert de fabrication de l'enveloppe. Cette conception permet un module qui soit particulièrement compact ;

Dans la présente demande, le terme « filtre » désigne des éléments de filtration disposés en aval des pompes pour protéger les organes moteur (injecteurs, enceintes) avec une filtration de l'ordre de 10 à 150 µm. La filtration des particules en amont des pompes est effectuée quant à elle par une « crépine » disposant d'un « treillis » empêchant les particules les plus grosses (supérieure à une taille de l'ordre de 500 à 1000 µm) d'abîmer les pompes.

### Avantages apportés

Les différentes solutions techniques du module d'attraction et de détection, à savoir une forme en H, plusieurs paires de pôles ou un aimant « oblique » avec un axe N-S incliné par rapport à l'axe de la forme cylindrique de l'aimant sont trois solutions qui permettent d'augmenter l'attraction des particules vers le module.

En effet, les différentes conceptions proposées augmentent la zone d'interaction entre le volume d'huile et l'aimant sans augmenter l'encombrement de l'aimant (et donc sans augmenter les pertes de charge liées à la présence du module dans le flux).

Les divers aspects additionnels permettent encore d'améliorer cette attraction.

Attirer un maximum de particules permet d'obtenir une mesure précise et représentative de l'état réel du flux.

D'autres bénéfices sont apportés par les différentes conceptions proposées, comme notamment un encombrement faible et donc une faible perturbation de l'écoulement du flux d'huile d'une part et un faible poids d'autre part.

Lorsque l'enveloppe s'étend au travers de plusieurs canaux, il est possible de détecter indépendamment la présence de débris dans chacun des canaux et d'en déduire précisément les éléments mécaniques qui peuvent être source de ces débris.

### Brève description des dessins

La figure 1 représente une turbomachine axiale selon l'invention ;
La figure 2 illustre une vue isométrique du corps d'un groupe de lubrification ;
Les figures 3A et 3B montrent deux exemples de systèmes de détection de débris selon l'invention ;
Les figures 4A à 4D montrent quatre exemples de modes de réalisation du module de détection de débris ;
Les figures 5 et 6 montrent un module intégré à une crépine ;
Les figures 7 à 9 montrent trois exemples d'intégration du module dans un passage de fluide double-canaux ou triple-canaux ;
Les figures 10A à 10C illustrent trois types d'aimant ;
Les figures 11 à 18 illustrent différentes variantes d'un module de détection selon l'invention, avec un aimant et une bobine.

### Description des modes de réalisation

Dans la description qui va suivre, le terme « aimant » renvoie à un aimant permanent. L'écoulement du flux dans le passage au niveau de l'aimant se déroule selon une direction principale d'écoulement qui est transversale au module (perpendiculaire ou simplement sécante). L'amont et l'aval sont entendus en relation avec le sens d'écoulement du flux.

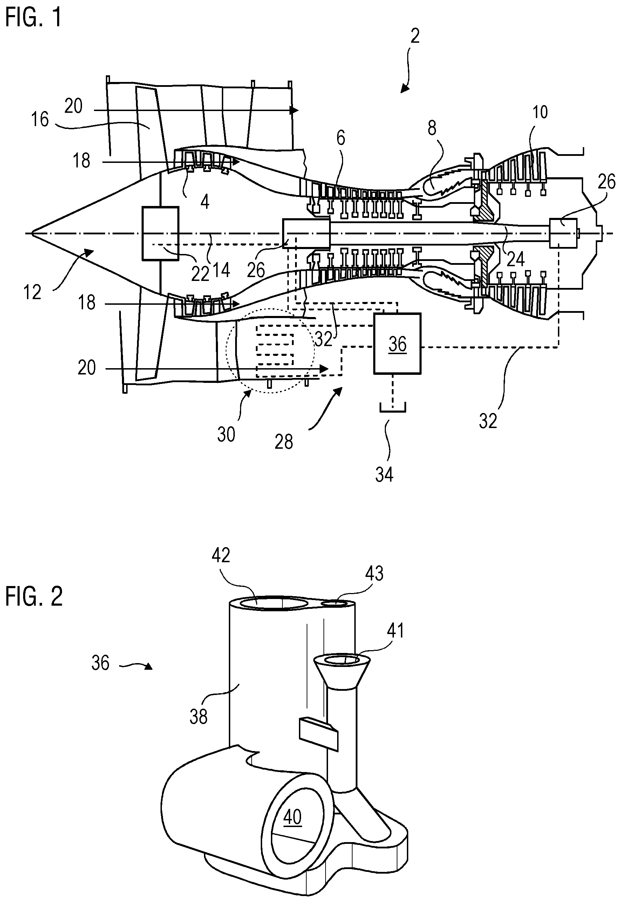

La figure 1 représente une turbomachine axiale ou turboréacteur double-flux. Le turboréacteur 2 comprend un compresseur basse-pression 4, un compresseur haute-pression 6, une chambre de combustion 8 et un ou plusieurs niveaux de turbines 10. En fonctionnement, la puissance mécanique des turbines 10 est transmise via des arbres jusqu'au rotor 12 et met en mouvement les deux compresseurs 4 et 6. La rotation du rotor autour de son axe de rotation 14 permet de générer un débit d'air et de comprimer progressivement ce dernier jusqu'à l'entrée dans la chambre de combustion 8.

Une soufflante 16 est couplée au rotor 12 et génère un flux d'air qui se divise en un flux primaire 18 traversant les différents niveaux susmentionnés de la turbomachine, et un flux secondaire 20 traversant un conduit annulaire. Des moyens de démultiplication 22 peuvent réduire la vitesse de rotation de la soufflante 16 et/ou du compresseur basse pression 4 par rapport à la vitesse de la turbine 10 associée.

Le rotor 12 comporte plusieurs arbres 24 coaxiaux supportés par des paliers 26. Le refroidissement et/ou la lubrification des paliers 26 et de l'optionnel réducteur 22 sont assurés par un circuit de lubrification 28. Le circuit de lubrification 28 peut comprendre un échangeur de chaleur 30 pour refroidir l'huile dont la température peut dépasser 200°C.

Le circuit de lubrification 28 peut comprendre des conduites 32 de récupération d'huile collectant l'huile dans les enceintes de lubrification des paliers 26 et l'acheminant dans le réservoir 34. Il peut également comporter une conduite 32 de récupération de l'huile lubrifiant le réducteur 22 et retournant cette huile dans le réservoir 34.

Afin de forcer la circulation de l'huile lors de sa récupération, le circuit de lubrification 28 peut comprendre un groupe de lubrification 36. Le groupe de lubrification 36 est une unité composée d'un corps monobloc qui accueille plusieurs fonctions hydrauliques comme par exemples plusieurs pompes et filtres. Il met en pression l'huile prélevée dans le réservoir et la distribue dans les organes du moteur à lubrifier avant de la reconditionner (refroidir et filtrer) et de la renvoyer vers le réservoir 34.

La figure 2 illustre un exemple en vue isométrique d'un corps 38 de groupe de lubrification 36. Le corps peut être fabriqué par fabrication additive et être de forme particulièrement complexe. Le corps 38 peut être monobloc. Il peut comporter plusieurs entrées d'huile 40, 42 pour aspirer l'huile depuis le réservoir ou depuis les organes de la turbomachine et plusieurs sorties d'huile 41, 43 pour refouler l'huile vers le réservoir ou vers les organes de la turbomachine. Des passages respectifs relient les entrées aux sorties. Certains passages peuvent être complètement indépendants d'autres passages. Le groupe 36 peut être équipé de nombreuses fonctions et contenir plusieurs pompes et plusieurs filtres. Selon l'invention, le groupe 36 peut aussi contenir un système de détection de débris ferromagnétiques.

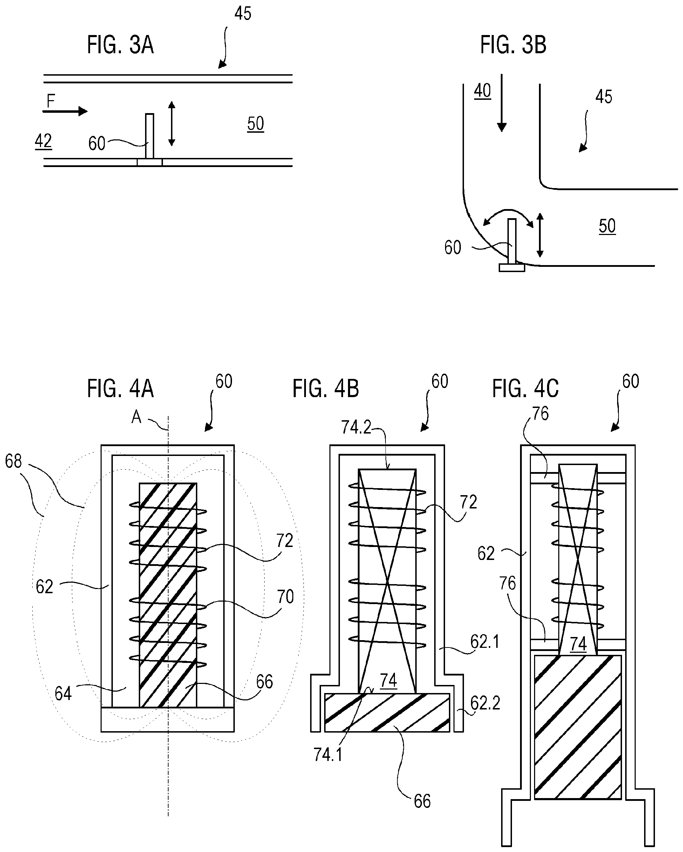

Les figures 3A et 3B schématisent deux versions d'un système de détection de débris 45 selon l'invention. Sur la figure 3A, un passage rectiligne 50, par exemple au voisinage de l'entrée 42, accueille un module d'attraction et de détection 60 des particules ferromagnétiques. Celui-ci vient en saillie dans le passage 50 et la longueur dont il vient en saillie peut être ajustable.

Sur la figure 3B, un passage coudé 50 par exemple au voisinage de l'entrée 40, accueille un module d'attraction et de détection 60. Celui-ci peut avoir une orientation par rapport au flux qui est ajustable et/ou une longueur en saillie dans le passage 50 qui est ajustable.

Le passage 50 est parcouru par un flux d'huile F. L'orientation et la longueur avec laquelle le module vient en saillie dans le passage sont ajustées mécaniquement par des moyens appropriés (moteur électrique, vis, piston, etc.).

Chaque système de détection 45 permet de détecter la présence et/ou la circulation de débris ferromagnétiques, ou particules ferromagnétiques, contenus dans l'huile. Ces débris peuvent notamment résulter d'une usure d'un palier ou de l'usure d'une dent d'engrenage du réducteur 22.

Chaque module 60 peut être connecté à une unité de traitement du signal (non représentée). Dès lors, l'unité de traitement parvient à identifier la présence de débris au niveau de chaque conduite. Les débris peuvent avoir une taille comprise entre 50 µm et 1000 µm, ou entre 150 µm et 750 µm.

Les figures 4A à 4C montrent trois exemples de module de détection 60. Le module 60 comprend une enveloppe 62 qui peut être cylindrique et d'axe A. L'enveloppe 62 délimite une cavité 64 qui accueille différents composants. L'enveloppe 62 est étanche et peut être disposée dans le flux F sans laisser pénétrer l'huile à l'intérieur de la cavité 64. L'enveloppe peut être faite de matériau polymère de 1 mm d'épaisseur (+/- 0.2 mm). Alternativement, l'enveloppe peut être plus fine, faite d'un alliage de titane ou être en acier inoxydable, et avoir une épaisseur d'environ 0.1 mm (+/- 0.02 mm). L'enveloppe est faite d'un matériau amagnétique.

Le module est destiné à mesurer le champ magnétique dans le flux F et plus particulièrement les variations du champ magnétique résultant du passage ou de la présence de débris ferromagnétiques.

À cet effet, la cavité 64 renferme un aimant permanent 66. Cet aimant peut être du type SmCo (Samarium-Cobalt) et avoir des propriétés magnétiques stables de -54°C à 200°C. La direction Sud-Nord de l'aimant 66 est ici selon l'axe A. Un champ magnétique 68 est schématiquement illustré en traits interrompus. D'autres exemples d'aimants sont illustrés plus loin.

La cavité 64 comprend également une ou plusieurs bobines 70, 72. Chaque bobine 70, 72 peut être faite d'un enroulement de plusieurs centaines de spires de fil fin (par exemple environ 0.01 mm) avec des épissures locales pour assurer la robustesse du fil. L'aimant 66 a le double rôle d'attirer les débris ferromagnétiques se trouvant dans le flux d'huile et de générer un champ magnétique détectable par la bobine 72. La bobine 72, passive, permet de mesurer les variations du champ magnétique 68 créé par l'aimant. La bobine 70 peut être une bobine « Built-in test » permettant de générer un champ magnétique et de vérifier la réponse de la bobine 72, par exemple avant la mise en fonctionnement d'un turboréacteur. La bobine 70 n'est donc pas essentielle au fonctionnement du module de détection mais permet une vérification intégrée de son bon fonctionnement.

De manière générale, la technologie de détection employée est semblable par exemple à la technologie divulguée dans le document WO 2017/157855 A1 ou dans le document EP 3 363 518 A1.

Ainsi, lorsqu'une particule ferromagnétique arrive à proximité de l'enveloppe, elle modifie le champ magnétique 68 et crée des discontinuités dans l'intensité de la bobine passive 72. Lorsque le signal comprend un pic qui dépasse un seuil donné prédéfini, le module 60 reconnaît qu'une particule ferromagnétique est passée.

La figure 4B montre un second exemple de mode de réalisation du module 60. Dans cet exemple, l'enveloppe 62 comprend deux portions 62.1, 62.2, dont une portion 62.2 de diamètre supérieur à l'autre portion 62.1. Ce ressaut 62.2 facilite le montage du module 60 sur une paroi du passage 50.

Dans l'exemple de la figure 4B, le module de détection 60 comprend un élément magnétique 74 sous forme de barreau magnétique (par exemple M50). Cet élément magnétique 74 peut traverser la ou les bobine(s) 70, 72. Il est au contact de l'aimant 66 au niveau de son extrémité proximale 74.1. Le barreau 74 se comporte ainsi comme le prolongement de l'aimant 66. Le champ magnétique est maximum à l'extrémité distale 74.2 du barreau 74. Le barreau 74 a préférentiellement un diamètre plus petit que l'aimant 66. L'aimant 66, le barreau 74 et la bobine 72 sont dimensionnés pour attirer et/ou détecter des particules de dimensions définies. Un aimant 66 trop puissant peut attirer de nombreuses particules mais génèrera un champ magnétique trop important et dont les variations seront difficilement décelables par la bobine 72. Un équilibre est donc trouvé.

La figure 4C illustre un troisième exemple de mode de réalisation du module 60. Cet exemple est sensiblement similaire à celui de la figure 4B. Des entretoises annulaires 76 sont prévues pour maintenir en position le barreau dans l'enveloppe 62. Dans cet exemple, l'aimant peut ne pas être confiné au ressaut 62.2.

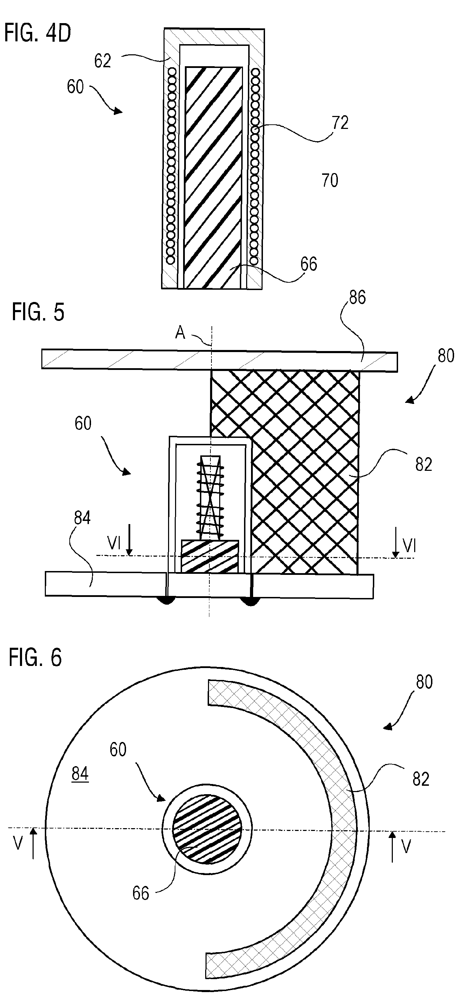

La figure 4D montre un exemple supplémentaire. Dans cet exemple la ou les bobines 70, 72, sont noyées dans l'enveloppe polymère, tels des inserts de fabrication. Pour ce faire, un fil de cuivre est enroulé et le polymère est injecté ou moulé autour du fil.

La figure 5 montre une première implémentation du module 60 dans une crépine 80. La crépine comprend un treillis de filtration 82 s'étendant d'une base 84 vers un plafond 86. Le module 60 peut être soudé à la base 84 de la crépine 80. Alternativement, un montage serré ou sertis peut être utilisé. Le treillis 82 peut prendre la forme d'un cylindre ou d'une portion de cylindre, par exemple s'étendant sur 180° autour de l'axe A. Le treillis 82 et le module 60 sont avantageusement coaxiaux. La figure 6 illustre ces aspects dans une section en vue en coupe selon l'axe VI:VI de la figure 5.

La taille des mailles du treillis 82 peut être supérieure ou égale à 1000 µm, ou à 750 µm, afin notamment de protéger les pompes en aval de la crépine des plus gros débris.

La crépine 80 peut être intégralement faite, y compris avec son treillis 82, par fabrication additive.

La figure 6 montre également l'ordre de grandeur du ratio entre le diamètre du module 60 et celui du treillis 82 qui peut être de l'ordre de 3.

L'ensemble de la figure 5 formé du module 60 et de la crépine 80 peut être inséré dans un passage 50 d'huile.

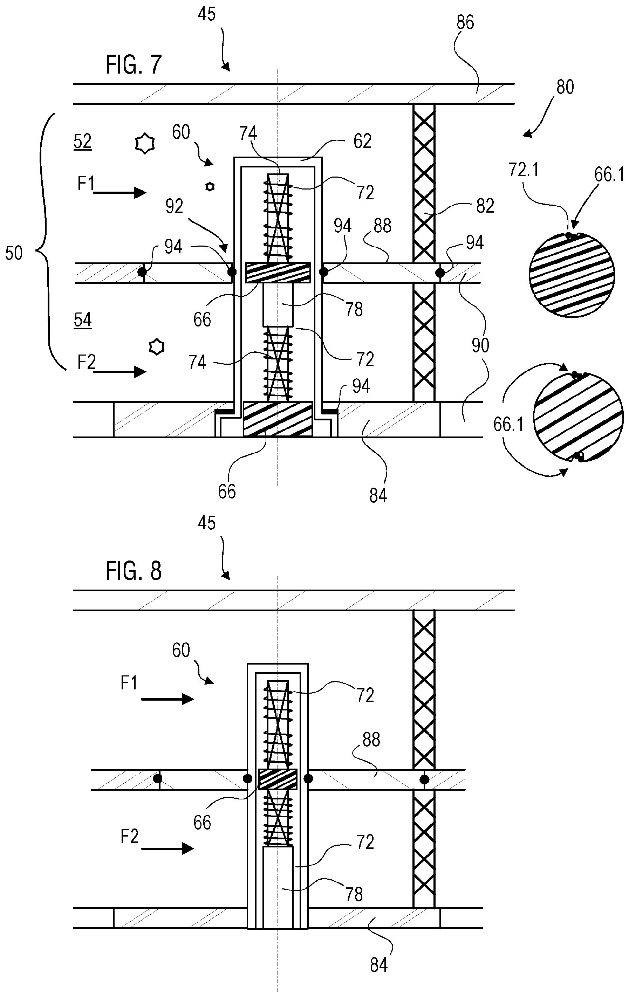

La crépine 80 peut également être multi-étages, avec une paroi intermédiaire entre la base 84 et le plafond 86. La figure 7 illustre une implémentation d'un module 60 avec une telle crépine 80 à deux étages dans un passage comprenant deux canaux 52, 54 dans lesquels circulent deux flux d'huile indépendants F1, F2. Dans cet exemple, le module 60 comprend deux aimants 66, deux barreaux magnétiques 74 et deux paires de bobines 70,72. Un bloc 78 en matériau isolant magnétique, par exemple un polymère, vient isoler le champ magnétique d'un aimant du champ magnétique de l'autre aimant, pour que les bobines 72 mesurent un champ magnétique issu de leur aimant 66 respectif sans interférence de l'autre aimant 66. Ainsi, un seul module 60 permet de détecter les particules ferromagnétiques de deux flux indépendants F1, F2 et donc d'en déduire de façon distinctive l'organe du turboréacteur qui émet des particules ferromagnétiques. Les aimants 66, les barreaux 74 et les bobines 72 dédiés aux deux canaux 52, 54 peuvent être différents. En effet, il peut être avantageux de prévoir des seuils de détection de particules qui soient différents selon les organes qui sont reliés respectivement à chaque canal 52, 54. Alternativement, ou en complément, la distinction des seuils de détection peut se faire sur le traitement des signaux provenant des deux bobines 72.

Dans cet exemple, la crépine 80 vient affleurer les parois 90 des canaux 52, 54. Des joints d'étanchéité 94 peuvent être prévus entre le module et la paroi intermédiaire 88 de la crépine 80, ainsi qu'entre la crépine 80 et les parois 90. Un orifice 92 dans la paroi 90 est prévu pour recevoir le module 60 et la crépine 80.

Cette figure illustre également un montage alternatif du module 60 dans la crépine, via le ressaut (62.2 sur la figure 4B). Le module peut être inséré axialement contre un joint d'étanchéité 94.

Sur la droite de la figure 7 sont illustrés les aimants 66 en coupe. Les aimants 66 disposent d'une rainure longitudinale 66.1 (selon l'axe A) qui permet le passage des câbles 72.1 des bobines 72. L'aimant supérieur (dans le sens de la figure 7) peut avoir une rainure 66.1 et l'aimant inférieur qui doit permettre le passage de plus nombreux fils peut avoir deux rainures 66.1.

La figure 8 illustre une alternative au système de la figure 7. Dans ce cas, un seul aimant 66 génère un flux magnétique pour les deux canaux 52, 54. L'aimant peut être confiné à la paroi intermédiaire 88.

Cette figure illustre également le fait que l'enveloppe 62 du module 60 peut être cylindrique sans ressaut et être sertie ou montée serrée dans la base 84 de la crépine.

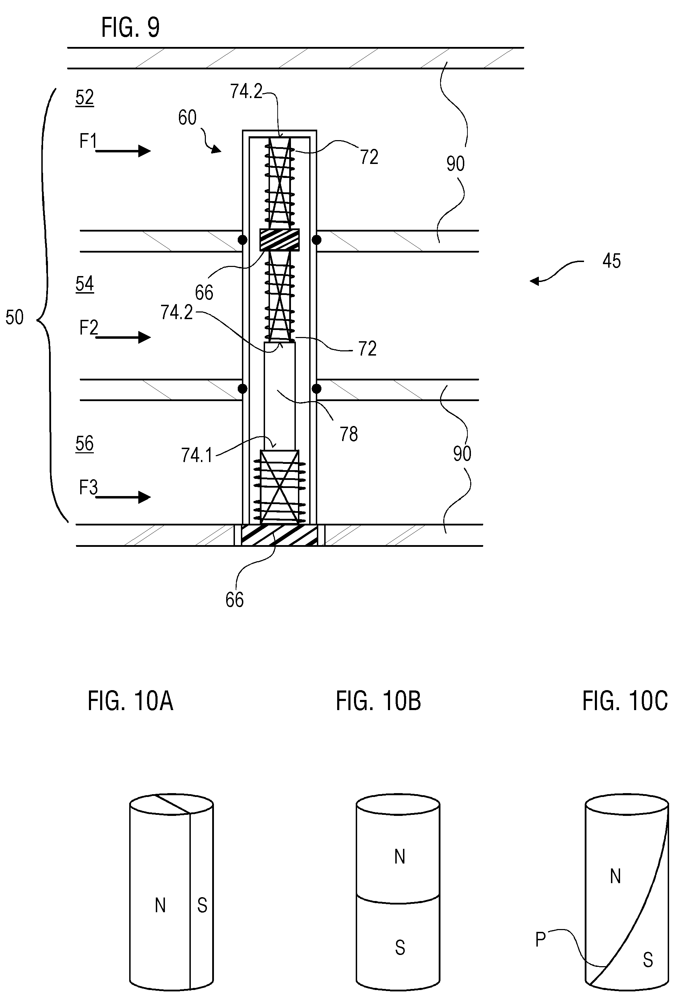

La figure 9 montre un exemple de module 60 permettant la mesure dans trois canaux 52, 54, 56 contigus deux à deux, parcourus par les flux respectifs F1, F2 et F3. Dans cet exemple, la crépine 80 qui reste optionnelle n'est pas représentée. On notera bien que la crépine n'est pas essentielle pour la mesure distinctive des particules dans plusieurs canaux indépendants. Le module 60 peut être hybride, c'est-à-dire présenter un aimant pour deux canaux 52, 54 et un aimant pour le troisième canal 56. Alternativement, trois aimants peuvent être prévus, avec trois barreaux magnétiques séparés deux à deux d'un bloc isolant. Le nombre de bobines 72 reste quant à lui toujours égal au nombre de canaux 52, 54, 56.

De manière générale, l'extrémité distale 74.2 de chaque barreau sera située aux environs du point médian du canal correspondant pour attirer et détecter un maximum de particules. La bobine de détection 72 correspondante sera également placée au voisinage du point médian des canaux. Par « point médian », on entend le milieu de la section du canal, situé entre ¼ et ¾ de la section du canal.

L'homme du métier comprendra que les détails techniques de chaque mode de réalisation sont applicables aux autres modes de réalisation. Notamment, le montage du module dans la crépine, la présence ou non d'un barreau, d'un ressaut, d'une bobine BIT**,** ou la présence d'une crépine sont des aspects optionnels et peuvent être tirés d'un mode de réalisation et appliqués à un autre.

Les figures 10A à 10C montrent trois types d'aimant.

L'aimant de la figure 10A génère un champ magnétique dont les lignes de champ sont perpendiculaires à l'axe de l'aimant.

L'aimant de la figure 10B produit un champ magnétique tel que dessiné sur la figure 4A. Cet aimant présente par contre un plus faible pouvoir d'attraction des particules.

L'aimant de la figure 10C présente deux pôles séparés par un plan qui est incliné par rapport à l'axe de l'aimant cylindrique. Le plan P n'est ni perpendiculaire ni parallèle à l'axe de l'aimant. Cette représentation est schématique. L'aimant peut être fait de plus petits aimants élémentaires permettant d'obtenir un aimant qui schématiquement comprend deux pôles opposés selon un tronçon de cylindre. Un tel aimant permet de bien attirer les particules mais également de créer un champ magnétique dont les variations sont détectables par une bobine coaxiale à l'aimant. L'angle entre le plan P et l'axe du cylindre peut être compris préférentiellement entre 60° et 80°.

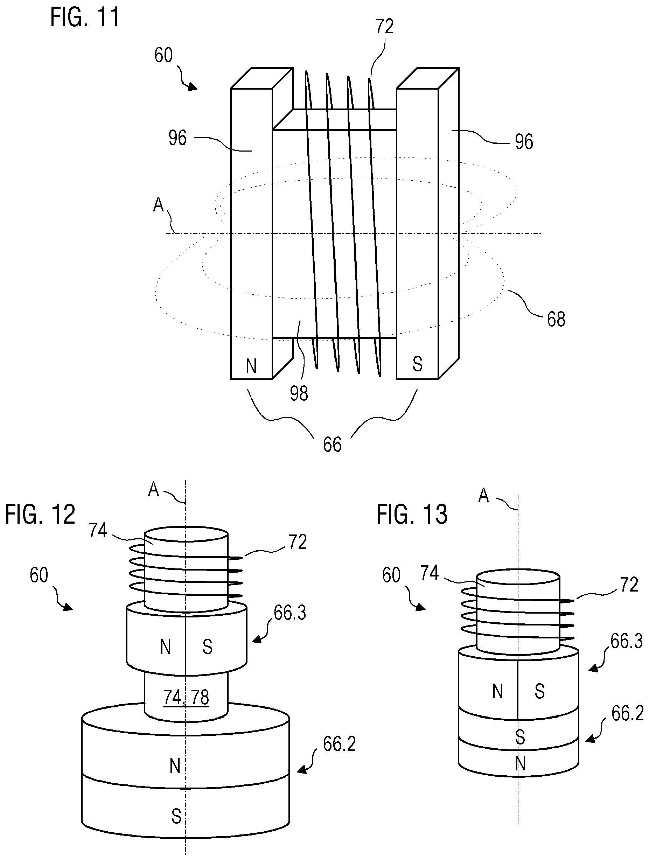

Comme l'illustrent les figures 11 à 18, ces trois types d'aimant des figures 10A à 10C peuvent être utilisés seuls ou en combinaison les uns des autres. Toutes ces solutions conduisent à des zones de plus grande attractivité pour les particules ferromagnétiques en comparaison d'un aimant standard utilisé seul.

La figure 11 décrit un mode de réalisation avec un module 6 de forme générale en H. Deux branches latérales 96 du H forment un aimant et la branche centrale 98 du H est entourée de la bobine 72. Le champ magnétique 68 est illustré en traits pointillés. L'axe A est l'axe d'enroulement de la bobine 72 mais également un axe sur lequel sont agencés les pôles de l'aimant.

La branche centrale 98 peut être partiellement ou complètement faite de fer doux ou être constituée en partie ou en totalité de samarium cobalt.

Dans une variante non représentée, les branches 96 peuvent chacune comprendre deux pôles, et plus particulièrement, vu de gauche à droite sur la figure 11, un pôle + et un pôle - sur la branche 96 de gauche, puis un pôle + et un pôle - sur la branche 96 de droite.

La figure 12 montre un autre exemple, dans lequel l'aimant est composé de deux paires de pôles 66.2, 66.3 distinctes et espacées par un tronçon de barreau. Les deux paires de pôles sont de deux types différents (en rapport aux exemples des figures 10A à 10C) et sont ici choisies avec l'option de présenter des diamètres différents.

Sur l'exemple de la figure 13, les deux paires de pôles sont adjacentes et de même diamètre.

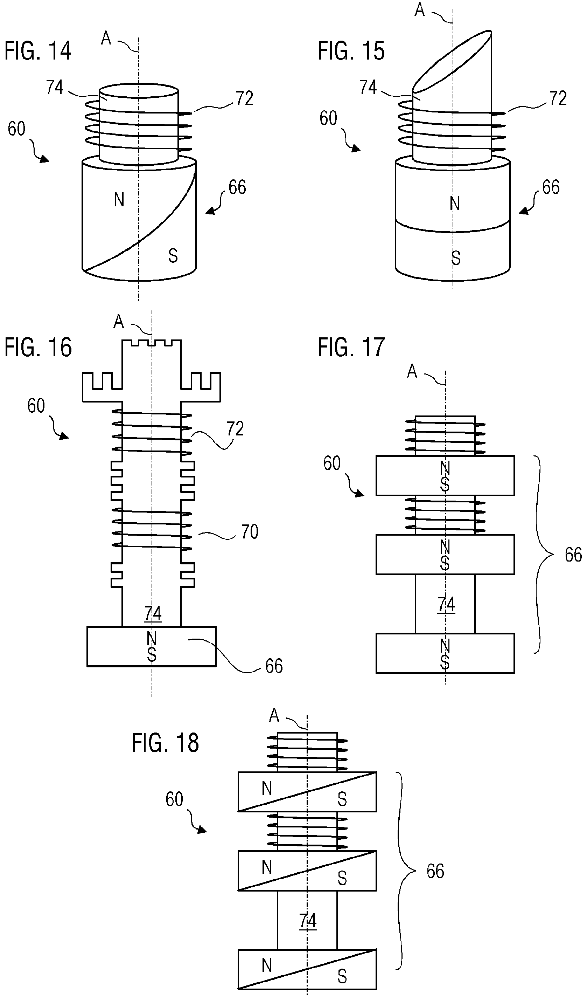

La figure 14 montre le cas d'un aimant oblique (cf. Fig. 10C).

La figure 15 illustre un exemple avec le barreau biseauté en son extrémité. Cette géométrie agrandit la surface externe du barreau.

La figure 16 montre en coupe un barreau « crénelé » c'est-à-dire avec des gorges et des variations de diamètre. Cette géométrie permet également d'agrandir le ratio de la surface du barreau sur son volume et donc d'agrandir le pouvoir d'attraction pour un même encombrement du barreau.

Alternativement ou en combinaison des exemples des figures 15 et 16, une surface externe du barreau striée peut également être prévue.

Enfin, les figures 17 et 18 montrent un aimant fait de trois paires de pôles, qui peuvent être identiques ou distinctes.

## Revendications

1. Module (60) d'attraction et de détection de débris ferromagnétiques dans un flux d'huile (F, F1, F2, F3) d'une turbomachine (2), le module (60) comprenant :
- un aimant permanent (66) ; et
- une bobine (72) apte à détecter le champ magnétique (68) généré par l'aimant (66) ;
le module (60) étant **caractérisé en ce que** l'aimant (66) dispose d'une géométrie en H, définissant une branche centrale (98) et deux branches latérales (96), la bobine (72) étant enroulée autour de la branche centrale (98) et les branches latérales (96) formant chacune un des pôles (N, S) de l'aimant (66).

2. Module (60) selon la revendication 1, **caractérisé en ce que** la branche centrale (98) est faite de fer doux ou de samarium-cobalt.

3. Module (60) d'attraction et de détection de débris ferromagnétiques dans un flux d'huile (F, F1, F2, F3) d'une turbomachine (2), le module comprenant :
- un aimant permanent (66) ; et
- une bobine (72) apte à détecter le champ magnétique (68) généré par l'aimant (66) ;
**caractérisé en ce que** l'aimant (66) comprend plusieurs paires de pôles (66.2, 66.3) et **en ce que** les paires de pôles (66.2, 66.3) sont agencées sur l'axe (A) de la bobine (72) et séparées les unes des autres par un séparateur ferreux (74) ou non ferreux (78).

4. Module (60) selon la revendication 3, **caractérisé en ce que** la bobine (72) est enroulée en partie autour du séparateur (74, 78).

5. Module (60) selon la revendication 4, **caractérisé en ce que** l'aimant (66) dispose d'une géométrie en H, définissant une branche centrale (98) et deux branches latérales (96), la bobine (72) étant enroulée autour de la branche centrale (98) et les branches latérales (96) formant chacune une paire de pôles (N, S).

6. Module (60) selon l'une des revendications 3 à 5, **caractérisé en ce que** les paires de pôles (66.2, 66.3) sont toutes identiques, présentant chacune deux pôles (N, S) axialement opposés, deux pôles (N, S) diamétralement opposés, ou deux pôles (N, S) opposés dans une direction qui n'est ni axiale ni diamétrale.

7. Module (60) selon l'une des revendications 3 à 6, **caractérisé en ce que** les paires de pôles (66.2, 66.3) comprennent au moins deux paires de pôles distinctes, choisies parmi les paires de pôles présentant deux pôles (N, S) axialement opposés, deux pôles (N, S) diamétralement opposés, ou deux pôles (N, S) opposés dans une direction qui n'est ni axiale ni diamétrale.

8. Module (60) d'attraction et de détection de débris ferromagnétiques dans un flux d'huile (F, F1, F2, F3) d'une turbomachine (2), le module (60) comprenant :
- un aimant permanent (66) ; et
- une bobine (72) apte à détecter le champ magnétique (68) généré par l'aimant (66) ;
**caractérisé en ce que** l'aimant (66) est de forme cylindrique avec deux pôles (N, S) séparés par un plan (P) qui n'est ni parallèle à l'axe (A) du cylindre, ni qui y est perpendiculaire.

9. Module (60) selon l'une des revendications 3 à 8, **caractérisé en ce qu'**il comprend en outre un barreau magnétique (74) au contact de l'aimant (66) et autour duquel est enroulée la bobine (72).

10. Module (60) selon la revendication 9, **caractérisé en ce que** le barreau (74) est fait de plusieurs portions de barreau, intercalées entre deux paires de pôles (66.2, 66.3) ; et/ou le barreau (74) a une extrémité distale (74.2) biseautée.

11. Module (60) selon l'une des revendications 9 ou 10, **caractérisé en ce que** le barreau (74) présente une surface striée ou crénelée, le barreau (74) ayant notamment des tronçons de différents diamètres.

12. Système (45) de détection de débris ferromagnétiques dans un flux d'huile (F, F1, F2, F3) d'une turbomachine (2), le système comprenant un passage (50) destiné à être parcouru par le flux (F, F1, F2, F3) et un module d'attraction et de détection (60) des débris ferromagnétiques présents dans le flux (F, F1, F2, F3), **caractérisé en ce que** le module (60) est selon l'une des revendications 1 à 11, est agencé dans le passage (50) et comprend en outre une enveloppe (62) étanche au flux (F, F1, F2, F3) dans laquelle sont confinés l'aimant (66) et la bobine (72).

13. Turboréacteur (2) d'aéronef comprenant un groupe de lubrification (36) fait d'un corps monobloc (38) recevant plusieurs pompes et filtres, plusieurs entrées et sorties d'huile (40-43), et un système de détection de débris, **caractérisé en ce que** le système (45) de détection des débris est selon la revendication 12 et le système (45) de détection des débris est disposé dans une entrée d'huile (40, 42) en amont des pompes et des filtres.

## Patentansprüche

1. Modul (60) zum Anziehen und Detektieren von ferromagnetischen Partikeln in einem Ölstrom (F, F1, F2, F3) einer Turbomaschine (2), wobei das Modul (60) umfasst :
- einen Permanentmagneten (66); und
- eine Spule (72), die dazu geeignet ist, das vom Magneten (66) erzeugte Magnetfeld (68) zu detektieren ;
wobei sich das Modul (60) dadurch auszeichnet, dass der Magnet (66) eine H-förmige Geometrie aufweist, die einen Mittelsteg (98) und zwei Seitenstege (96) definiert, dass die Spule (72) um den Mittelsteg (98) gewickelt ist und dass die Seitenstege (96) jeweils einen der Pole (N, S) des Magneten (66) bilden.

2. Modul (60) nach Anspruch 1, **dadurch gekennzeichnet, dass** der Mittelsteg (98) aus Weicheisen oder aus Samarium-Kobalt besteht.

3. Modul (60) zum Anziehen und Detektieren von ferromagnetischen Partikeln in einem Ölstrom (F, F1, F2, F3) einer Turbomaschine (2), wobei das Modul umfasst :
- einen Permanentmagneten (66); und
- eine Spule (72), die dazu geeignet ist, das vom Magneten (66) erzeugte Magnetfeld (68) zu detektieren ;
**dadurch gekennzeichnet, dass** der Magnet (66) mehrere Polpaare (66.2, 66.3) umfasst und dass die Polpaare (66.2, 66.3) auf der Achse (A) der Spule (72) angeordnet und voneinander durch einen eisenhaltigen (74) oder einen nicht eisenhaltigen (78) Separator getrennt sind.

4. Modul (60) nach Anspruch 3, **dadurch gekennzeichnet, dass** die Spule (72) teilweise um den Separator (74, 78) gewickelt ist.

5. Modul (60) nach Anspruch 4, **dadurch gekennzeichnet, dass** der Magnet (66) eine H-förmige Geometrie aufweist, die einen Mittelsteg (98) und zwei Seitenstege (96) definiert, dass die Spule (72) um den Mittelsteg (98) gewickelt ist und dass die Seitenstege (96) jeweils ein Polpaar (N, S) bilden.

6. Modul (60) nach einem der Ansprüche 3 bis 5, **dadurch gekennzeichnet, dass** die Polpaare (66.2, 66.3) alle identisch sind und jeweils zwei axial zueinander entgegengesetzte Pole (N, S), zwei diametral zueinander entgegengesetzte Pole (N, S) oder zwei in einer Richtung, die weder axial noch diametral ist, zueinander entgegengesetzte Pole (N, S) aufweisen.

7. Modul (60) nach einem der Ansprüche 3 bis 6, **dadurch gekennzeichnet, dass** die Polpaare (66.2, 66.3) mindestens zwei unterschiedliche Polpaare umfassen, ausgewählt aus den Polpaaren mit zwei axial zueinander entgegengesetzten Polen (N, S), zwei diametral zueinander entgegengesetzten Polen (N, S) oder zwei in einer Richtung, die weder axial noch diametral ist, zueinander entgegengesetzten Polen (N, S).

8. Modul (60) zum Anziehen und Detektieren von ferromagnetischen Partikeln in einem Ölstrom (F, F1, F2, F3) einer Turbomaschine (2), wobei das Modul (60) umfasst :
- einen Permanentmagneten (66); und
- eine Spule (72), die dazu geeignet ist, das vom Magneten (66) erzeugte Magnetfeld (68) zu detektieren ;
**dadurch gekennzeichnet, dass** der Magnet (66) zylindrisch geformt ist und zwei Pole (N, S) aufweist, die durch eine Ebene (P) getrennt sind, die weder parallel zur Achse (A) des Zylinders noch senkrecht zu dieser Achse (A) ist.

9. Modul (60) nach einem der Ansprüche 3 bis 8, **dadurch gekennzeichnet, dass** es ferner einen magnetischen Stab (74) umfasst, der mit dem Magneten (66) in Kontakt steht und um den die Spule (72) gewickelt ist.

10. Modul (60) nach Anspruch 9, **dadurch gekennzeichnet, dass** der Stab (74) aus mehreren Stababschnitten besteht, die zwischen zwei Polpaaren (66.2, 66.3) angeordnet sind, und/oder dass der Stab (74) ein abgeschrägtes distales Ende (74.2) aufweist.

11. Modul (60) nach einem der Ansprüche 9 oder 10, **dadurch gekennzeichnet, dass** der Stab (74) eine gerillte oder gekerbte Oberfläche aufweist, wobei der Stab (74) insbesondere Abschnitte mit unterschiedlichen Durchmessern aufweist.

12. System (45) zur Detektion ferromagnetischer Partikel in einem Ölstrom (F, F1, F2, F3) einer Turbomaschine (2), wobei das System einen Durchgang (50), der dazu bestimmt ist, vom Strom (F, F1, F2, F3) durchströmt zu werden, und ein Modul (60) zum Anziehen und Detektieren der im Strom (F, F1, F2, F3) vorhandenen ferromagnetischen Partikel umfasst, **dadurch gekennzeichnet, dass** das Modul (60) nach einem der Ansprüche 1 bis 11 ausgebildet ist, in dem Durchgang (50) angeordnet ist und ferner eine gegenüber dem Strom (F, F1, F2, F3) dichte Hülle (62) umfasst, in welcher der Magnet (66) und die Spule (72) eingeschlossen sind.

13. Flugzeugturbostrahltriebwerk (2), das eine Schmiergruppe (36) umfasst, die aus einem einstückigen Gehäuse (38) besteht, das mehrere Pumpen und Filter, mehrere Öl-Einlässe und -Auslässe (40-43) sowie ein Partikeldetektionssystem aufnimmt, **dadurch gekennzeichnet, dass** das Partikeldetektionssystem (45) nach Anspruch 12 ausgebildet ist und das Partikeldetektionssystem (45) in einem Öleinlass (40, 42) stromaufwärts der Pumpen und Filter angeordnet ist.

## Claims

1. A module (60) for attracting and detecting ferromagnetic debris in an oil flow (F, F1, F2, F3) of a turbomachine (2), the module (60) comprising :
- a permanent magnet (66); and
- a coil (72) configured to detect the magnetic field (68) generated by the magnet (66) ;
the module (60) being **characterized in that** the magnet (66) has an H-shaped geometry, defining a central branch (98) and two lateral branches (96), the coil (72) being wound around the central branch (98) and the lateral branches (96) each forming one of the poles (N, S) of the magnet (66).

2. The module (60) according to claim 1, **characterized in that** the central branch (98) is made of soft iron or samarium-cobalt.

3. A module (60) for attracting and detecting ferromagnetic debris in an oil flow (F, F1, F2, F3) of a turbomachine (2), the module comprising :
- a permanent magnet (66); and
- a coil (72) configured to detect the magnetic field (68) generated by the magnet (66) ;
**characterized in that** the magnet (66) comprises a plurality of pairs of poles (66.2, 66.3) and **in that** the pairs of poles (66.2, 66.3) are arranged along the axis (A) of the coil (72) and are separated from one another by a ferrous separator (74) or a nonferrous separator (78).

4. The module (60) according to claim 3, **characterized in that** the coil (72) is wound in part around the separator (74, 78).

5. The module (60) according to claim 4, **characterized in that** the magnet (66) has an H-shaped geometry, defining a central branch (98) and two lateral branches (96), the coil (72) being wound around the central branch (98) and the lateral branches (96) each forming a pair of poles (N, S).

6. The module (60) according to any one of claims 3 to 5, **characterized in that** the pairs of poles (66.2, 66.3) are all identical, each having two poles (N, S) that are axially opposed, two poles (N, S) that are diametrically opposed, or two poles (N, S) that are opposed in a direction that is neither axial nor diametrical.

7. The module (60) according to any one of claims 3 to 6, **characterized in that** the pairs of poles (66.2, 66.3) comprise at least two distinct pairs of poles, selected from the pairs of poles having two poles (N, S) that are axially opposed, two poles (N, S) that are diametrically opposed, or two poles (N, S) that are opposed in a direction that is neither axial nor diametrical.

8. A module (60) for attracting and detecting ferromagnetic debris in an oil flow (F, F1, F2, F3) of a turbomachine (2), the module (60) comprising :
- a permanent magnet (66); and
- a coil (72) configured to detect the magnetic field (68) generated by the magnet (66) ;
**characterized in that** the magnet (66) is cylindrical in shape with two poles (N, S) separated by a plane (P) which is neither parallel nor perpendicular to the axis (A) of the cylinder.

9. The module (60) according to any one of claims 3 to 8, **characterized in that** it further comprises a magnetic bar (74) in contact with the magnet (66) and around which the coil (72) is wound.

10. The module (60) according to claim 9, **characterized in that** the bar (74) is made of several bar portions interposed between two pairs of poles (66.2, 66.3); and/or the bar (74) has a bevelled distal end (74.2).

11. The module (60) according to any one of claims 9 or 10, **characterized in that** the bar (74) has a grooved or crenelated surface, the bar (74) in particular having sections of different diameters.

12. A system (45) for detecting ferromagnetic debris in an oil flow (F, F1, F2, F3) of a turbomachine (2), the system comprising a passage (50) intended to be traversed by the flow (F, F1, F2, F3) and a module (60) for attracting and detecting the ferromagnetic debris present in the flow (F, F1, F2, F3), **characterized in that** the module (60) is according to any one of claims 1 to 11, is arranged in the passage (50) and further comprises a casing (62) sealed against the flow (F, F1, F2, F3) in which the magnet (66) and the coil (72) are confined.

13. An aircraft turbojet engine (2) comprising a lubrication assembly (36) comprising a single-piece body (38) receiving a plurality of pumps and filters, a plurality of oil inlets and outlets (40-43), and a debris detection system, **characterized in that** the debris detection system (45) is according to claim 12 and the debris detection system (45) is arranged in an oil inlet (40, 42) upstream of the pumps and the filters.
